# EUROPEAN PATENT SPECIFICATION

(11) **EP 1 638 454 B1**
(45) Date of publication and mention of the grant of the patent: **22.06.2016**
(21) Application number: 04776189.5
(22) Date of filing: 28.05.2004
(51) Int. Cl.: A61B 5/145, A61B 5/15, A61B 5/151, A61B 5/155

(54) **APPARATUS FOR BODY FLUID SAMPLING AND ANALYTE SENSING**
GERÄT ZUR ENTNAHME VON KÖRPERFLÜSSIGKEITEN UND MESSUNGEN VON ANALYTEN
APPAREIL POUR L'ECHANTILLONNAGE DE LIQUIDE ORGANIQUE ET LA DETECTION D'ANALYTE

(30) Priority: 30.05.2003 US 452815; 30.05.2003 US 474915 P; 03.07.2003 US 613517
(43) Date of publication of application: 29.03.2006
(73) Proprietor: Sanofi-Aventis Deutschland GmbH, 65929 Frankfurt am Main (DE)
(72) Inventor: BOECKER, Dirk, Palo Alto, CA 94306 (US); FREEMAN, Dominique, M., La Honda, CA 94020 (US); ALDEN, Don, Sunnyvale, CA 94087 (US); SHUMANN, Matt, Cambridge CB3 7RY (GB); BEADMAN, Mike, Cambridge CB3 7RY (GB)
(74) Representative: McDougall, James
(86) International application number: PCT/US2004/017063
(87) International publication number: WO 2005/001418

(56) References cited:
- WO-A-97/42882
- WO-A-03/071940
- WO-A1-01/00090
- DE-C1- 10 057 832
- US-A- 6 093 156
- US-A1- 2002 087 056
- US-A1- 2003 083 685
- US-A1- 2004 039 303
- US-A1- 2004 230 216
- US-B1- 6 228 100

## Description

### BACKGROUND OF THE INVENTION

Lancing devices are known in the medical health-care products industry for piercing the skin to produce blood for analysis. Typically, a drop of blood for this type of analysis is obtained by making a small incision in the fingertip, creating a small wound, which generates a small blood droplet on the surface of the skin.

Early methods of lancing included piercing or slicing the skin with a needle or razor. Current methods utilize lancing devices that contain a multitude of spring, cam and mass actuators to drive the lancet. These include cantilever springs, diaphragms, coil springs, as well as gravity plumbs used to drive the lancet. The device may be held against the skin and mechanically triggered to ballistically launch the lancet. Unfortunately, the pain associated with each lancing event using known technology discourages patients from testing. In addition to vibratory stimulation of the skin as the driver impacts the end of a launcher stop, known spring based devices have the possibility of firing lancets that harmonically oscillate against the patient tissue, causing multiple strikes due to recoil. This recoil and multiple strikes of the lancet is one major impediment to patient compliance with a structured glucose monitoring regime.

Another impediment to patient compliance is the lack of spontaneous blood flow generated by known lancing technology. In addition to the pain as discussed above, a patient may need more than one lancing event to obtain a blood sample since spontaneous blood generation is unreliable using known lancing technology. Thus the pain is multiplied by the number of attempts required by a patient to successfully generate spontaneous blood flow. Different skin thickness may yield different results in terms of pain perception, blood yield and success rate of obtaining blood between different users of the lancing device. Known devices poorly account for these skin thickness variations.

A still further impediment to improved compliance with glucose monitoring are the many steps and inconvenience associated with each lancing event. Many diabetic patients that are insulin dependent may need to self-test for blood glucose levels five to six times daily. The large number of steps required in traditional methods of glucose testing, ranging from lancing, to milking of blood, applying blood to a test strip, and getting the measurements from the test strip, discourages many diabetic patients from testing their blood glucose levels as often as recommended. Older patients and those with deteriorating motor skills encounter difficulty loading lancets into launcher devices, transferring blood onto a test strip, or inserting thin test strips into slots on glucose measurement meters. Additionally, the wound channel left on the patient by known systems may also be of a size that discourages those who are active with their hands or who are worried about heaving of those wound channels from testing their glucose levels.

US 2003/083685 A1 discloses a tissue penetration device and method of using the same. The device optionally includes sampling and analysing functions. An example provides control of a lancet for sampling blood using electric field coils or solenoids to drive the lancet. Advancement and retraction of the lancet is controlled by a feedback loop monitoring the position and velocity of the lancet and may be configured to follow a predetermined tissue lancing profile.

DE 100 57 832 C1 discloses a blood analysis device having a syringe mounted in a casing and operated by an external plunger. An annular mounting carries needles, each of which is mounted behind a test strip. The mounting is swiveled so that a needle cooperates with the syringe and the needle can be pushed through the test strip and an aperture in the casing to take a blood sample.

US 2002/087056 A1 discloses an analyte monitoring device having a housing and comprising a plurality of needles, each having a tip, a retracted position, a position wherein the tip is extended from the housing a distance adapted to pierce skin, an electrically or spring powered needle pushing apparatus movable to separately engage each of the needles to move each from the retracted position to the extended position, an energy source located within the housing, a plurality of analysis sites comprising an analysis preparation, each adapted to receive liquid from the needles to wet the analysis preparation, one or more light sources adapted to direct light at the analysis sites, one or more light detectors adapted to receive light from the analysis sites, and a processor.

### SUMMARY OF THE INVENTION

The present invention is defined by the claims.

### BRIEF DESCRIPTION OF THE DRAWINGS

Figures 98 and 99 show schematic views of penetrating member drivers according to the present invention.
Figure 100 shows a penetrating member driver according to the present invention for use with a cartridge containing a plurality of penetrating members.
Figures 101 and 102 show a penetrating member driver using a magnetically controllable fluid device.

### DESCRIPTION OF THE SPECIFIC EMBODIMENTS

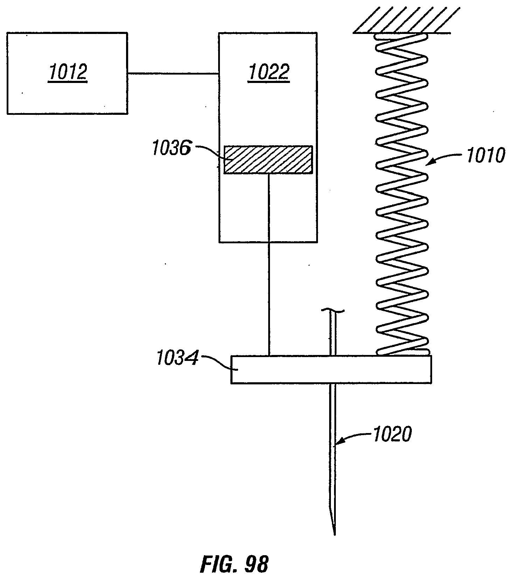

Referring now to Figure 98, an embodiment of an actuator according to the present invention will now be described. The present invention relates to an actuator 1010 that will launch a lancet or penetrating member 1020 into skin or an anatomical feature in a controlled manner so as to produce a small drop of blood or body fluid while minimizing patient discomfort. As a nonlimiting example, energy stored in a compressed spring, gas, or other actuation technique is released to actuate a lancet 1020. Through the use of processor 1012, the motion of the lancet or penetrating member 1020 is controlled by an iron-loaded fluid 1022 that changes viscosity in response to an imposed magnetic field. A motor or other device (not shown) may be used to control the retraction rate of the lancet 1020 from the skin or other targeted anatomical feature. It should be understood, of course, that other magnetically controllable fluid as known to those skilled in the art may also be used.

Figure 98 documents the concept of using a magnetic fluid to control the action of a mechanical spring. In the embodiment of Figure 98, energy is stored in the compressed spring and released at the time of actuation. As previously discussed, other actuators besides the compressed spring may also be used.

The motion of the lancet is controlled by means of an electromagnet that is arranged to produce a magnetic field in a fluid consisting of fine iron particles suspended in oil, silicone fluid, or other medium. When a magnetic field is imposed on the fluid, the iron particles align with the field, and resist motion. Fluid firmness increases with field strength. A suitable fluid can be purchased as MIRF-132AD Rheonetic Fluid from Lord Corporation (888) 811-5673.

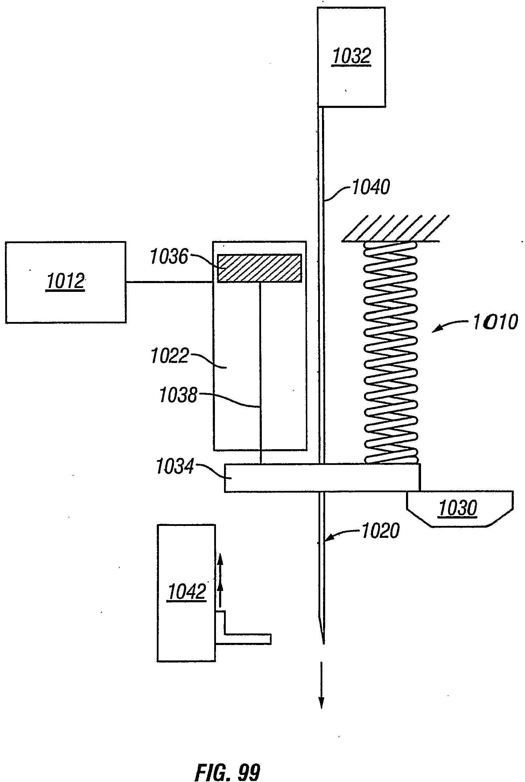

Figure 99 provide details about launching and resetting the actuator for the present embodiment. A firing catch 1030 is shown to hold the spring 1010 in a cocked position prior to firing. An optically reflective member such as a flag 1032 is shown attached to the lancet coupler 1034 to provide position feedback through an optical position transducer. In some embodiments, the flag 1032 may be attached to a drive shaft (not shown). This feedback allows a processor 1012 to modulate the current to the electromagnetic coil or other magnetic field generator as known to one skilled in the art, to control the actuation profile of the lancet. A disc 1036 is shown attached to the penetrating member coupler 1034 and the disc is submerged in the rheonetic fluid. Suitable seals may be used to contain the fluid while allowing the shaft 1038 to pass through the dashpot chamber. In some embodiments, the disc 1036 is mounted about shaft 1040 and the entire dashpot chamber is also mounted about a portion of the shaft 1040. A motor 1042, or other retraction device is shown to move the dashpot and carry the drive shaft back to the cocked position. The motor then resets the dashpot to the desired stop position, and the actuation cycle is ready to repeat.

One advantage of this design is that each actuator can be matched to a portion of the actuation cycle. Rapid energy release is provided by the spring 1010 to bring the lancet or penetrating member 1020 up to speed. In one embodiment, excess energy stored in the spring allows the actuator 1010 to maintain the desired lancet speed regardless of skin or tissue consistency. The rheonetic fluid 1022 in the dashpot, controlled by the electromagnet, dissipates the excess energy from the spring 1010. A DC reset motor 1042 can be driven at variable speeds by controlling the motor drive current. By this means, the retraction speed of the lancet can be controlled.

Another advantage of this present embodiment is that power consumption is reduced through the use of a small DC motor instead of a solenoid. The motor draws energy from a battery at a much lower rate and over a longer time, resulting in more efficient battery use.

In another aspect, the present embodiment provides a device for storing and rapidly releasing energy. The device controls the release of stored energy to control motion, controls the release of energy to provide a low impact stop, controls the storage of energy to control retraction motion, and stores energy for rapid release at the start of the next cycle.

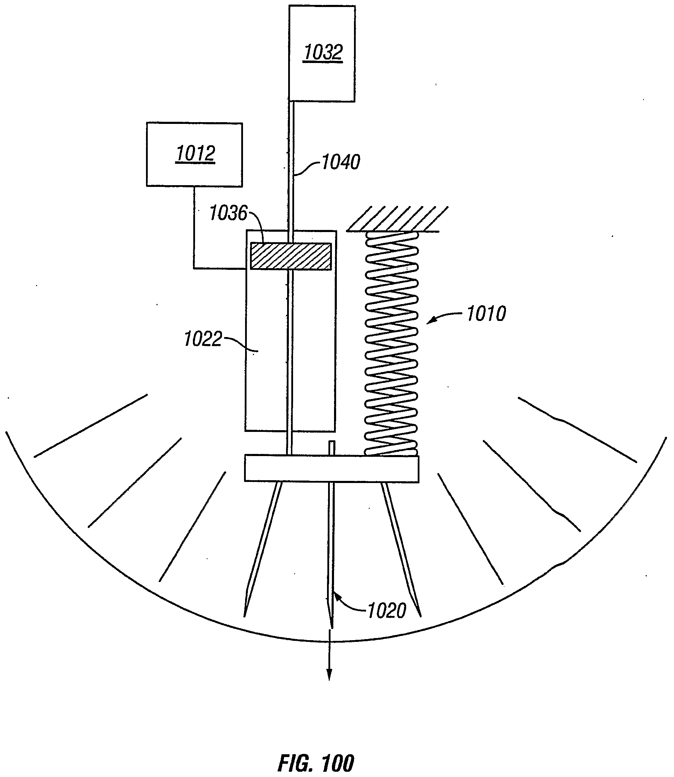

Figure 100 shows that embodiments of the lancet actuators of Figures 98 and 99 may be configured for use with a radial cartridge 1050 having a plurality of penetrating members 1020. Accordingly, these launchers may be coupled with single use or multiple use lancing devices. As a nonlimiting example, these devices may be used with a cartridge 500.

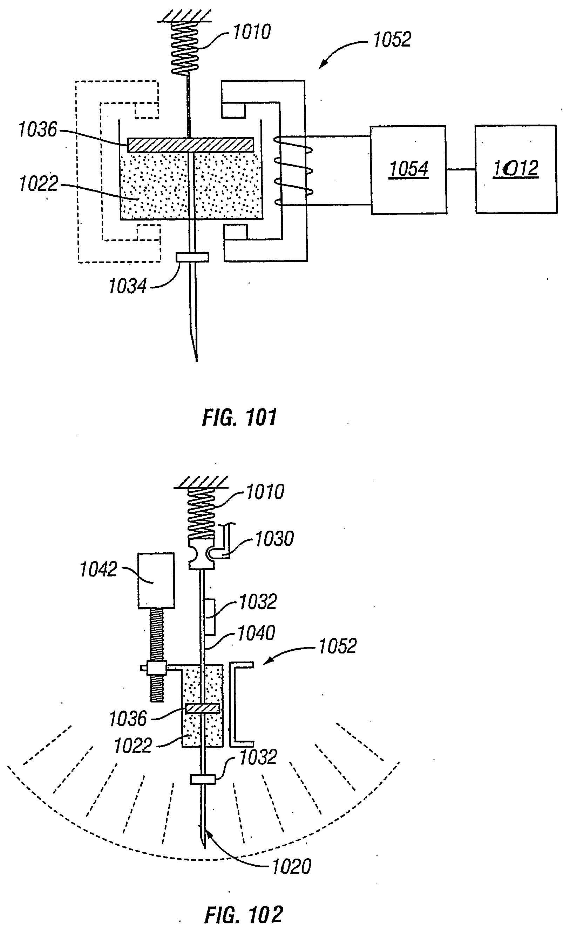

Figure 101 shows a more detailed view of one embodiment of an electromagnetic field generator 1052 coupled to a power source 1054 controlled by a processor 1012.

Figure 102 shows a still further embodiment similar to that shown in Figure 99. This embodiment includes an actuator 1010 (shown in this nonlimiting example to be a spring), a disc 1036 coaxially mounted about a shaft 1040 in a ferrofluid 1022, and a flag 1032 for monitoring lancet or penetrating member position. The launch device of Figure 102 may also be adapted for use with a radial cartridge (shown in phantom) having a plurality of penetrating members 1020 which may be coupled to the coupler 1034.

Referring still to Figure 102, energy is stored in the compressed spring used as actuator 1010 and is released at the time of actuation. In this embodiment, the motion of the penetrating member 1020 is controlled by an electromagnet 1052 that is arranged to produce a magnetic field in a fluid consisting of fine iron particles or other material suspended in but not limited to oil, silicone fluid, or other medium. When a magnetic field is imposed on the fluid, the iron particles align with the field, and resist motion. Fluid firmness increases with field strength. Such fluid can be purchased as MRF-132AD Rheonetic Fluid from Lord Corporation (888) 811-5673. A flag is shown attached to the drive shaft to provide position feedback through an optical position transducer. This feedback allows a processor to modulate the current to the electromagnetic coil to control the actuation profile of the lancet. A disc is shown attached to the drive shaft and submerged in the rheonetic fluid. Suitable seals are required to contain the fluid while allowing the shaft to pass through the dashpot chamber. A motor, or other driving device is shown to move the dashpot and carry the drive shaft back to the cocked position. The motor then resets the dashpot to the desired stop position, and the actuation cycle is ready to repeat. The advantage of this design is that each actuator may be matched to a portion of the actuation cycle. Rapid energy release is provided by the spring to bring the lancet up to speed. Excess energy stored in the spring allows the actuator to maintain the desired lancet speed regardless of skin consistency. The rheonetic fluid in the dashpot, controlled by the electromagnet, dissipates the excess energy from the spring. Of course, other dashpots or dampers as disclosed herein or as known to one of skill in the art may also be used. In one embodiment, a DC reset motor can be driven at variable speeds by controlling the motor drive current. By this motor, the retraction speed of the penetrating member 1020 can be controlled. A second advantage of this invention is that power consumption is reduced through the use of a small DC motor instead of a solenoid. The motor draws energy from a battery at a much lower rate and over a longer time, resulting in more efficient battery use. This hybrid device could also be configured to yield a "smart braking" pattern so that residual pain is minimized.

## Claims

1. A body fluid sampling system for use on a tissue site, the system comprising:
a cartridge (1050);
a penetrating member driver (1010);
a plurality of penetrating members (1020) arranged in a radial configuration on the cartridge (1050) wherein sharpened distal tips of the penetrating members (1020) point radially outward;
wherein an active one of said penetrating members (1020) is configured to be operatively coupled to said penetrating member driver (1010) ;
**characterized in** comprising an electromagnet arranged to control motion of the active one penetrating member (1020) by applying a magnetic field to an iron-loaded fluid (1022) that changes viscosity in response to the applied magnetic field; and
a processor (1012) configured to control the magnetic field applied to said iron-loaded fluid by said electromagnet.

2. A system as in claim 1, wherein said penetrating member driver comprises a spring (1010) based launching device.

## Patentansprüche

1. Körperflüssigkeitsprobenahmesystem zur Verwendung an einem Gewebeort, wobei das System Folgendes umfasst:
eine Kartusche (1050),
einen Penetrierelementtreiber (1010),
eine Vielzahl von Penetrierelementen (1020), die in einer radialen Konfiguration an der Kartusche (1050) angeordnet sind, wobei scharfe distale Spitzen der Penetrierelemente (1020) radial nach außen weisen,
wobei ein aktives der Penetrierelemente (1020) dazu konfiguriert ist, an den Penetrierelementtreiber (1010) wirkgekoppelt zu sein,
**dadurch gekennzeichnet, dass** es einen Elektromagneten umfasst, der zur Steuerung der Bewegung des aktiven Penetrierelements (1020) angeordnet ist, indem er ein Magnetfeld an ein eisenbeladenes Fluid (1022) anlegt, das als Reaktion auf das angelegte Magnetfeld seine Viskosität ändert, sowie einen Prozessor (1012), der zur Steuerung des durch den Elektromagneten an das eisenbeladene Fluid angelegten Magnetfelds konfiguriert ist.

2. System nach Anspruch 1, wobei der Penetrierelementtreiber eine auf einer Feder (1010) basierende Auslösevorrichtung umfasst.

## Revendications

1. Système d'échantillonnage de fluide corporel pour l'utilisation sur un site tissulaire, le système comprenant :
une cartouche (1050) ;
un dispositif d'entraînement d'élément de pénétration (1010) ;
une pluralité d'éléments de pénétration (1020) disposés dans une configuration radiale sur la cartouche (1050), des pointes distales aiguisées des éléments de pénétration (1020) étant orientées radialement vers l'extérieur ;
un élément de pénétration actif parmi lesdits éléments de pénétration (1020) étant configuré pour être accouplé fonctionnellement audit dispositif d'entraînement d'élément de pénétration (1010) ;
**caractérisé en ce qu'**il comprend un électroaimant prévu pour contrôler le mouvement de l'élément de pénétration actif (1020) en appliquant un champ magnétique à un fluide chargé de fer (1022) qui change de viscosité en réponse à l'application du champ magnétique ; et
un processeur (1012) configuré pour contrôler le champ magnétique appliqué audit fluide chargé de fer par ledit électroaimant.

2. Système selon la revendication 1, dans lequel ledit dispositif d'entraînement d'élément de pénétration comprend un dispositif de lancement à base d'un ressort (1010).
